# EUROPEAN PATENT APPLICATION

(11) **EP 1 911 491 A1**
(43) Date of publication of application: **16.04.2008**
(21) Application number: 07253732.7
(22) Date of filing: 20.09.2007
(51) Int. Cl.: A61N 1/36, A61H 39/00

(54) **Healthcare device for electrostimulation**

(30) Priority: 13.10.2006 GB 0620381
(71) Applicant: Immumax International Co., Ltd., Hong Kong (HK)
(72) Inventor: Wong, Shuk Ching Judy, Kowloon Hong Kong (HK)
(74) Representative: Chettle, Adrian John

(57) **Abstract**

Apparatus and method for addressing and alleviating health problems by applying pulse stimulation via electrode attached to the hand at specific point located along the second metacarpal bone of the hand linking with the index finger. The method includes an apparatus, a system and a scheme to identify the treatment point on the hand, applied in combination, will invoke the human immune system to achieve healing effect or improve symptoms of illness, particularly those caused by inflammation, e.g. asthma, hay fever and related allergies, sore throat and tooth ache.

## Description

### Technical Field

Embodiments of the invention relate generally to the field of bioscience. The invention includes an electronic pulse generation apparatus and a special method for addressing or alleviating healthcare problems or improving the associated symptoms by invoking the human immune system.

### Background to the Invention

Conventional electro-stimulation treatment for diseases requires a user to apply stimulation via an electrode in electrical contact with the body. Treatment is applied for duration ranging form minutes to hours for a prescribed period, depending on the disease nature and seriousness.

A conventional electro-stimulation system normally includes a pulse generation device and adhesive electrode pads for attachment to the body. Such systems are usually used for the relief of back pain or muscle stiffness. These systems fall under the general classification of "transcutaneous electrical nerve stimulation systems (TENS)."

Diseases, in particular chronic diseases, can be treated in many ways. One common method is by taking medicine or injection. Apart from cost, there is the question of allergy to medicine to certain patients, not to mention the adverse effect on some organs (e.g. kidney) because of prolonged medication.

In recent years, alternative treatment methods have become popular and for example, acupuncture and reflexology have been widely accepted in western countries to the extent that practitioners can register with government authorities. There are, however, disadvantages associated with these methods:
Acupuncture - Firstly, this method cannot be administered by the patients themselves, so they have to rely on the skill of the acupuncturists, which varies from person to person. Secondly, being punctured by needles is scary to some people, not to mention risks that may arise from mishandling of needles. Thirdly, this method is inconvenient to patients, as treatment must take place at pre-arranged time.

Reflexology, acupuncture and acupressure - All of these methods have to be administered by practicing experts at pre-arranged time and place. This would mean such treatment may not be always available if one travels abroad. Moreover, the pain generated by these treatment methods is very intense in some cases. As a result some patients are forced to discontinue such treatment.

Electro Stimulation Methods - Many pulse current generation devices are available in the market. The more common ones are devices designed for application to ear, hand or to muscle. However, there are limitations or problems associated with such devices; for example, the most common type of pulse generation devices which have adhesive electrode pads can only treat back pain or produce muscle relaxation effects. There are also known cases of temporary loss of memory caused by the sudden surge in pulse intensity generated by ear devices. As for hand devices, the common problem is the difficulty in identifying the right spot for effective treatment.

### Summary of the Invention

The invention aims to provide an apparatus, a system and a method to address or alleviate healthcare problems via electro-stimulation by invoking the healing effect of the human immune system. The invention also aims at improving some problems associated with electro-stimulation treatment.

According to the invention there is provided an apparatus comprising: a portable device for generating electrical pulse stimulation; and having an external electrode which is adapted to be applied to a treatment point on the hand, over the side of the second metacarpal bone. Preferably the device is pre-programmed with a plurality of treatment regimes, and in the preferred embodiment two electrodes are provided, one for application to each hand.

The invention also comprises a method of use comprising use of the apparatus of claim 1 to address health problems by application of pulsed stimulation to a treatment point over the second metacarpal bone. The device is applied to the external surface of the skin, at a point immediately over and at the side of the second metacarpal bone.

Embodiments of the invention are intended to introduce an additional alternative healthcare apparatus which is safe, portable and convenient to use. Features of the invention will be apparent from the appended claims.

The system according to one embodiment of the invention includes specially designed functions which extend such use to children aged 5 and above.

An embodiment of the invention includes a specially designed electrode with round-head point, which is couched in soft material such as plastic, and made in the form of a belt. The belt is applied according to instructions to a selected treatment point, to provide a quick and reliable remedy to health problems.

The method according to the invention is to apply treatment only on the hand irrespective of the location of the ailment. This means that diseases in different parts of the body can be treated on the hand.

### Brief Description of the Drawings

The invention and advantageous features are explained in more detail with reference to the accompanying drawings in which:
Fig. 1 is a front-view schematic diagram representing an apparatus and a system according to preferred embodiments of the invention.
Fig. 2 is a simplified block diagram representing a system according to embodiments of the invention.
Fig. 3 is a diagram representing an article of the preferred embodiment of the invention, which forms part of the apparatus and system.
Fig. 4 is a diagram showing a set of treatment points located at the side of the second metacarpal bone in relation to different parts of the body.
Fig. 5 is a diagram illustrating the application of an electrode belt to a treatment point on the hand, according to an embodiment of the invention.
Fig. 6 is a flow diagram illustrating a method according to an embodiment of the invention.

### Detailed Description

In the following description of various embodiments of the invention, information with respect to these embodiments, including a best mode of practising such embodiments, is provided. Reference is made to the drawings which form a part hereof, and in which are shown by way of illustration, and not of limitation to how the invention may be practised.

The embodiments illustrated are described in sufficient detail to enable those skilled in the art to practise them. Other embodiments may be utilized and derived therefrom, such that electronic, structural and other changes may be made without departing from the scope of the claims. The following detailed description is provided in a nonlimiting sense, and variations are of course possible within a range of equivalents which such claims encompass.

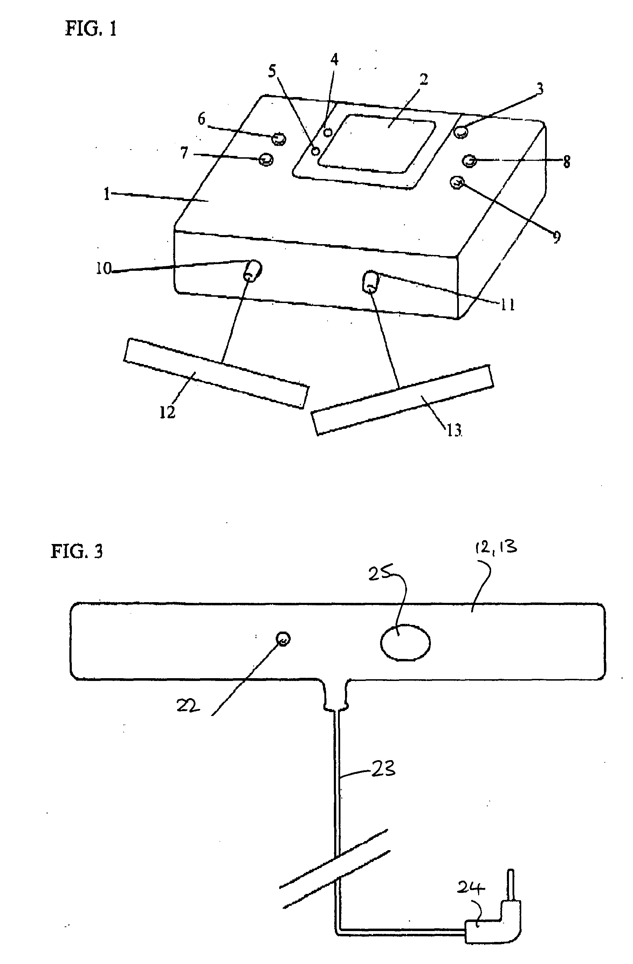

Fig. 1 shows the representational front view of an apparatus and a system comprising the characteristics of the invention. It includes: the housing (1), preferably of plastic material; a Liquid Crystal Display (2) to show operation mode, operation time (including the running time) and pulse intensity level; a Power On/Off Button (3); a Mode Selection Button (4) to provide a choice between Adult and Child functions; a Time Selection Button (5) to control operation time (e.g. 15, 30, 45 minutes); two Intensity Control ( + ) Buttons (6 and 8) to increase pulse intensity level; two Intensity Control (-) Buttons (7 and 9) to decrease pulse intensity level; two Output Connections (10 and 11) for corresponding electrode belts, and which are inserted to channel pulse current; and two electrode belts (12 and 13) to conduct pulse stimulation to the contact points on the hands of the user.

Preferably, the apparatus is small, such that it is convenient to use, and to carry; the apparatus is thus suitable for use whilst travelling.

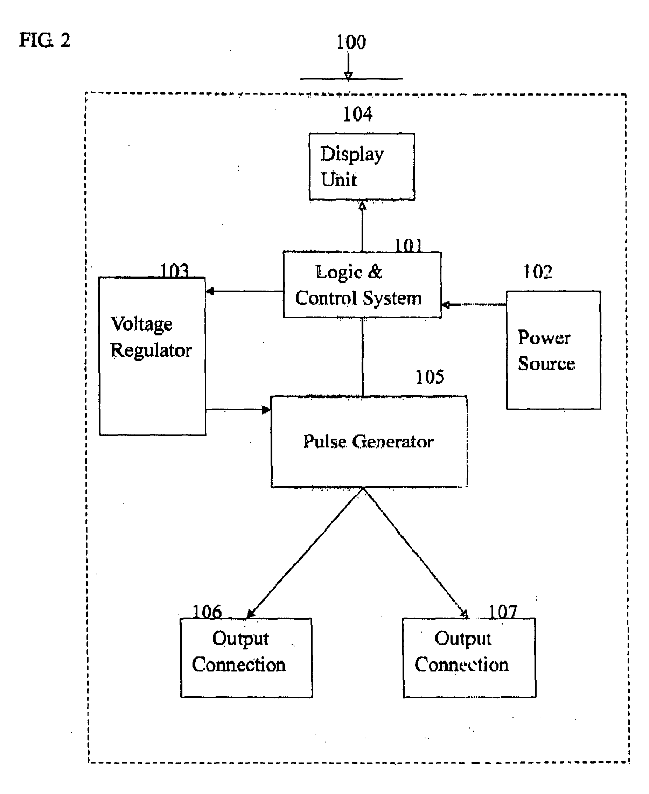

Fig. 2 is a block diagram of a system according to various embodiments of the invention. The apparatus embodies the system (100), comprising: The Logic and Control System (101), which is set in a central processing unit and has at least four modules:
• An Operation Mode Module to device and control the Adult/Child functions
• A Pulsation Module - to drive and control pulse intensity and frequency levels
• An Operation Time Module - to drive and control selection of different periods of treatment time
• A display Module - to control the information to be displayed in the LCD

When the Power Button (3) is pressed, 101 power is supplied to the system 101 from an internal battery or chip (102), typically a replaceable 9V dry cell is provided.

When the Mode Selection Button (4) is pressed, the Operation Mode Module and the Display Module of the system 101 are activated. These modules generate signals for the Display Unit (104), which will show indication of Adult/Child program for the user to select.

Once the selection is made, the Operation Mode Module will accordingly trigger the preset program of the Pulsation Module of system 101, which will pass signals to the Voltage Regulator (103), which will adjust the voltage according to the signals received to make the Pulse Generator (105) ready to generate pulse stimulation.

When the Intensity Control Buttons (6 to 9) are pressed, the Pulsation Module and the Display Module of system 101 are activated. The Pulsation Module will pass signals to regulator 103 to adjust the voltage as necessary to enable generator 105 to generate pulse stimulation of the selected intensity and frequency and output the pulse current via the two output connections (106 and 107). The Display Module will trigger the Display Unit to show the selected intensity level. Frequency of pulsing is in the range 1-10 Hertz, typically 2-3 Hertz. The intensity level is variable in the range 0.5 mA to 10 mA.

When the Time Selection Button (5) is pressed, the Operation Time Module and Display Module of system 101 are activated. Pre-programmed periods of operation, e.g. 15, 30, 45 minutes will appear on the Display Unit. Once a period is selected, the Operation Time Module will trigger the internal clock to count time and instruct the Display Module to show the running time on the Display Unit. When the selected period is up, the Operation Time Module will trigger the system to shut down.

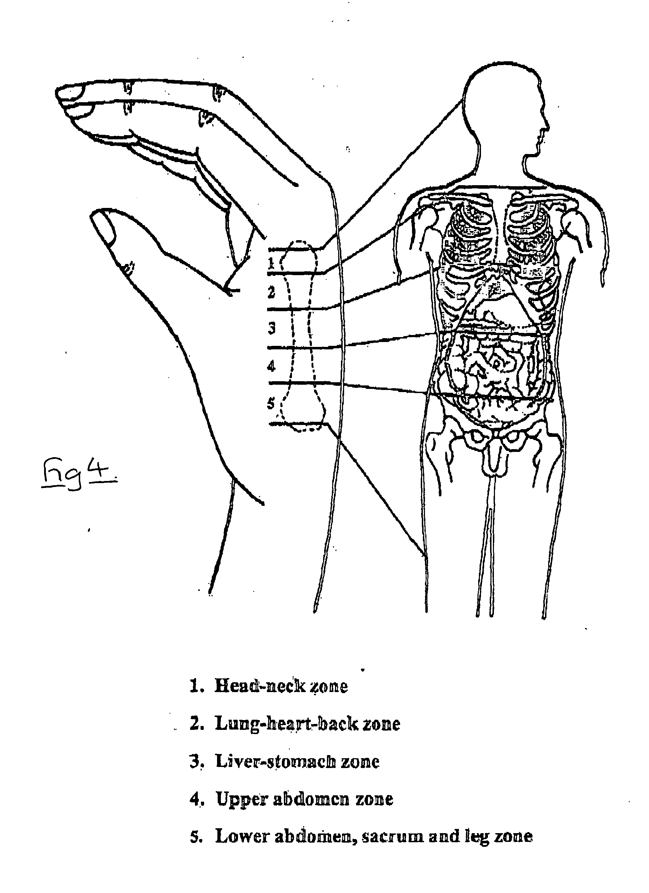

Fig. 3 shows a belt 12,13 for the hand, and a special electrode (22) which form part of the apparatus and system of the invention. The electrode has a small round-head point as its anode which is set on a soft fabric belt of plastic material. The round-head anode is specially designed, so it can press firmly against a treatment point on the hand to channel the pulse stimulation.

The electrode belt has a twin core wire (23) and at its end is a plug (24) which is inserted into one of the output connections (106 and 107) to channel pulse current via a contact point on the user's hand. A suitable adjustable strap (e.g. using hook and loop fasteners) allows the belt to be fastened firmly around the hand). A left hand and right hand belt are provided.

The electrode 22 is a generally smooth rounded projection about 3mm in diameter and arranged centrally in a belt of about 33mm width. The electrode protrudes from the surface of the belt by about 3mm. An earthing terminal 25 completes an electrical circuit through the user and comprises a generally flat metal disc about 16mm in diameter and about 2mm thick. The electrode 22 and earthing terminal 25 are about 60mm apart. The dimensions of the belt 12,13, electrode 22 and earthing terminal 25 are selected for comfort and effectiveness.

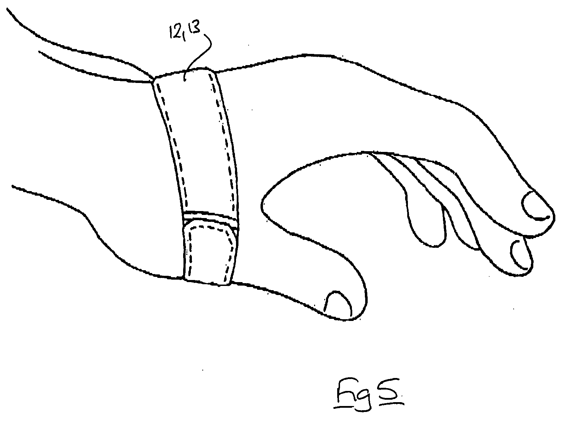

Fig. 4 based on the research of a Chinese scholar, the late Professor Zhang Yinqing, the nerves and cell groups at the side of the second metacarpal bone (31) of the hand, linking with the index finger represent a replica of the whole body. These nerves and cell groups are schematically located along the said bone and are related to different parts of the body as illustrated in Fig. 4. If a part of the body is disturbed or is not well, the corresponding nerves and cell groups at the side of the second metacarpal bone on the hand are affected and will have a symptom; for example a sore point will be formed. By applying pulse current to this sore point on the hand, the immune system is stimulated and a self-healing power is released to address the relevant point on the metacarpal bone, and this effect is carried to the corresponding part of the body. In this way, a health problem is cured or alleviated. The sore point of the metacarpal bone is the treatment point. Stimulation is for a period typically in the range 15-45 minutes, depending on the nature and seriousness of the health problem.

The metacarpal bone is preferably divided into five zones, which correspond to five parts of the body as indicated in Fig. 4. A greater number of zones may be adopted if desired.

Fig. 5 illustrates how the electrode belt 12,13 with the round-head anode is applied to the hand. A lot of research and clinical experience in recent years have proved that many diseases can be addressed by the human immune system instead of taking conventional medical treatment. One advantage of the invention is that it provides an additional user-friendly and effective alternative or natural way to protect health and to alleviate health problems.

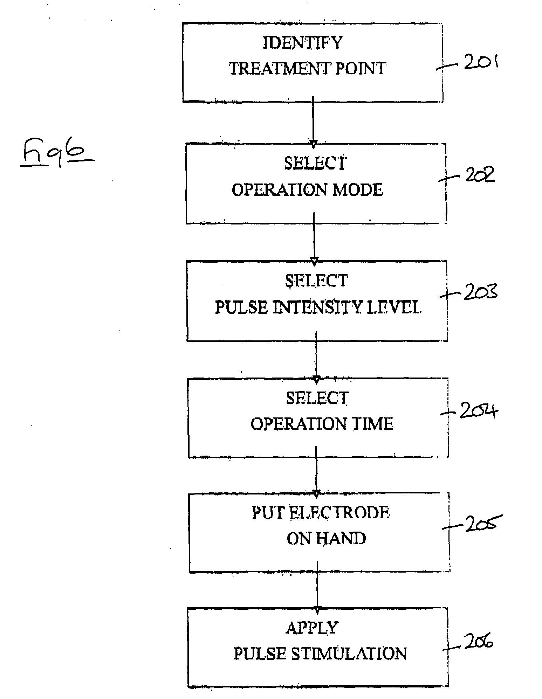

Fig. 6 provides a flow diagram of the method of a preferred embodiment of the invention. The flow diagram in Fig. 7 shows the steps of applying the apparatus and method of the invention.

Step 201 relates to the identification of the treatment point by pressing gently on the side of the second metacarpal bone on the hand which is linked with the index finger to locate the sore point. This step is repeated for both hands.

Step 202, 203 and 204 relates to the operation of the apparatus to select the Adult or Child function; to select the pulse intensity level; and to select the treatment time.

Step 205 relates to putting the round-head anode against the sore point as the treatment point.

Step 206 relates to the electrode is put in contact of the hand and pulse current is channelled to the contact point, i.e. the treatment point.

The invention is not restricted to the details of the foregoing embodiments. The invention extends to any novel one or novel combination, of the features disclosed (including claims, abstract and drawings), or to any novel one, or any novel combination, of the steps of any method or process so described.

## Claims

1. An apparatus comprising: a portable device for generating electrical pulse stimulation; and having an external electrode which is adapted to be applied to a treatment point on the hand, over the side of the second metacarpal bone.

2. The apparatus according to claim 1, and comprising a palm-size control unit adapted to be powered by an internal battery and having a belt for attachment to a hand, said belt including said electrode for application at the second metacarpal bone.

3. The apparatus according to claim 1 or claim 2, wherein said electrode is within soft plastic material made into the form of a belt for wrapping around the hand.

4. The apparatus according to claim 3, wherein the electrode is in the form of a round-head point adapted to transmit pulse current to said treatment point.

5. The apparatus of any preceding claim, and having two electrodes for respective application to the left and right hands.

6. The apparatus according to claim 5, and having two output connections for channelling pulse current to respective electrodes for the left and right hands.

7. The apparatus of any preceding claim, being pre-programmed and including a Liquid Crystal Display Unit to show programmed functions.

8. The apparatus of any preceding claim and incorporating a Logic and Control System, comprising a Central Processing Unit with programs to drive and control the operation mode, operating time, pulse generation and pulse intensity thereof.

9. The apparatus of claim 8, and including a first function specially designed for application to children aged 5 and above, and a second function for adults.

10. The apparatus according to claim 9, wherein the pulse intensity for children is different from the pulse intensity for adults.
